# EUROPÄISCHE PATENTSCHRIFT

(11) **EP 1 131 040 B1**
(45) Veröffentlichungstag und Bekanntmachung des Hinweises auf die Patenterteilung: **15.12.2004**
(21) Anmeldenummer: 99971303.5
(22) Anmeldetag: 27.10.1999
(51) Int. Cl.: A61K 7/06, A61K 7/32

(54) **ETHANOLISCHE CHITOSAN ENTHALTENDE KOSMETISCHE ZUBEREITUNGEN**
ETHANOLIC COSMETIC PREPARATIONS CONTAINING CHITOSAN
PREPARATIONS COSMETIQUES ETHANOLIQUES CONTENANT DE LA CHITOSANE

(30) Priorität: 04.11.1998 DE 19850734
(43) Veröffentlichungstag der Anmeldung: 12.09.2001
(73) Patentinhaber: Cognis Deutschland GmbH & Co. KG, 40589 Düsseldorf (DE)
(72) Erfinder: PANZER, Claudia, D-41515 Grevenbroich (DE); TESMANN, Holger, D-41363 Jüchen (DE); WACHTER, Rolf, D-40595 Düsseldorf (DE)
(86) Internationale Anmeldenummer: PCT/EP1999/008105
(87) Internationale Veröffentlichungsnummer: WO 2000/025734

(56) Entgegenhaltungen:
- EP-A- 0 803 513
- WO-A-87/07618
- DE-C- 19 701 417
- US-A- 5 690 921
- US-A- 5 690 924
- PATENT ABSTRACTS OF JAPAN vol. 1998, no. 10, 31. August 1998 (1998-08-31) & JP 10 114603 A (DAINIPPON JOCHUGIKU CO LTD), 6. Mai 1998 (1998-05-06)
- PATENT ABSTRACTS OF JAPAN vol. 014, no. 014 (C-674), 12. Januar 1990 (1990-01-12) & JP 01 256375 A (KIMITSU KAGAKU KENKYUSHO:KK), 12. Oktober 1989 (1989-10-12)

## Beschreibung

### Gebiet der Erfindung

Die Erfindung befindet sich auf dem Gebiet der Kosmetik und betrifft Formulierungen mit hohem Ethanolgehalt und speziellen Chitosansalzen.

### Stand der Technik

Chitosane stellen wegen ihrer feuchtigkeitsspendenden und filmbildenden Eigenschaften wertvolle Rohstoffe für die Herstellung von kosmetischen Mitteln dar. Da die Chitosane selbst nicht wasseriöslich sind, werden sie üblicherweise in einer kosmetisch akzeptablen Säure, vorzugsweise Glycolsäure gelöst und in dieser Zubereitungsform in den Handel gebracht. Von Nachteil ist dabei jedoch, daß übliche im Handel erhältliche Chitosanlösungen eine nicht ausreichend hohe Alkoholverträglichkeit aufweisen und daher für die Herstellung von versprühbaren kosmetischen Mitteln mit hohem Ethanolgehalt, wie beispielsweise Haarsprays oder Deoformulierungen nur eingeschränkt geeignet sind.

In diesem Zusammenhang sei auf die Europäische Patentanmeldung **EP 0368253 A2** (Union Carbide) verwiesen, aus der pharmazeutische Zubereitungen zur zeitverzögerten Abgabe von Wirkstoffen bekannt sind, welche Neutralisationsprodukte von Chitosan mit den verschiedensten Carbonsäuren enthalten.

Die Aufgabe der vorliegenden Erfindung hat daher darin bestanden, neue kosmetische Mittel zur Verfügung zu stellen, die Chitosane in einer besonders alkoholverträglichen Anbietungsform enthalten.

### Beschreibung der Erfindung

Gegenstand der Erfindung sind alkoholische kosmetische Zubereitungen, enthaltend
(a) 70 bis 90 Gew.-% Ethanol und
(b) 0,01 bis 5 Gew.-% Neutralisationsprodukte von Chitosan mit Pyrrolidoncarbonsäure, Nicotinsäure, Hydroxyisobuttersäure, Hydroxyisovaleriansäure und deren Gemischen,
mit der Maßgabe, daß sich die Mengenangaben mit Wasser sowie gegebenenfalls weiteren Hilfs- und Zusatzstoffen zu 100 Gew.-% ergänzen. Werden die Zubereitungen mit Hilfe von Treibgasen versprüht, so kann das Gewichtsverhältnis der alkoholischen Zubereitungen zu den Treibgasen 35 : 65 bis 45 : 55 betragen.

Überraschenderweise wurde gefunden, daß die speziellen Chitosansalze sich durch eine stark verbesserte Ethanolverträglichkeit auszeichnen und so nunmehr die Herstellung von überwiegend alkoholbasierten Formulierungen, wie z.B. Haarsprays, Haargelen und Deoformulierungen erlauben. Auf diesem Wege werden Haarsprays erhalten, die sich gegenüber den Produkten des Stands der Technik durch eine deutlich gesteigerte festigende Wirkung auszeichnen. Im Bereich der Haargele werden je nach Einsatzmenge der Chitosane bis zu schnittfeste Zubereitungen erhalten, so daß auf die Mitverwendung von Verdickungsmitteln verzichtet werden kann. Im Bereich der Aerosolformulierungen können Mittel hergestellt werden, die eine vergleichsweise höhere Hautverträglichkeit besitzen und zudem die Haare nicht belasten, dafür aber Kopfschuppen und Splißbildung entgegenwirken.

### Chitosansalze

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten: 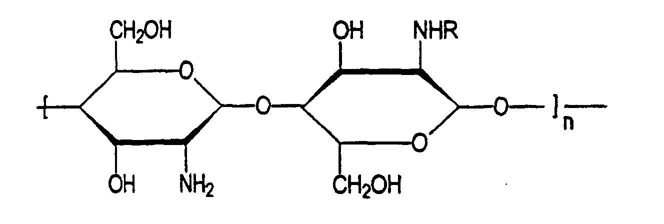 Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332).** Übersichten zu diesem Thema sind auch beispielsweise von B.Gesslein et al. in **HAPPl 27**, **57 (1990),** O.Skaugrud in **Drug Cosm.Ind**. **148, 24 (1991)** und E.Onsoyen et al. in **Seifen-Öle-Fette-Wachse 117**, **633 (1991)** erschienen. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Entsprechende Verfahren sind beispielsweise aus **Makromol.Chem. 177, 3589 (1976)** oder der französischen Patentanmeldung **FR 2701266 A1** bekannt. Vorzugsweise werden solche Typen eingesetzt, wie sie in den deutschen Patentanmeldungen **DE 4442987 A1** und **DE 19537001 A1** (Henkel) offenbart werden, und die ein durchschnittliches Molekulargewicht entweder von 10.000 bis 1.000.000 oder von 100.000 bis 5.000.000 Dalton, eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% aufweisen. Die Chitosane werden in 1 bis 10, vorzugsweise 2 bis 5 Gew.-%iger Verdünnung in Pyrrolidoncarbonsäure, Nicotinsäure, Hydroxyisobuttersäure, Hydroxyisovaleriansäure und deren Gemischen eingesetzt und liegen dann als Salze, nämlich als Pyrrolidoncarboxylate, Nicotinate, Hydroxybutyrate und/oder Hydroxyisovaleriate vor.

### Gewerbliche Anwendbarkeit

Die erfindungsgemäßen Zubereitungen, bei denen es sich vorzugsweise um versprühbare Produkte wie Haarbehandlungsmittel (speziell Haarsprays) oder Deoformulierungen und daneben Gele handelt, können als weitere Hilfs- und Zusatzstoffe milde Tenside, Ölkörper, Emulgatoren, Überfettungsmittel, Perlglanzwachse, Stabilisatoren, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Lichtschutzfilter, insektenrepellentien, Selbstbräuner, Treibgase, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv® TN), Dialkylether, Ringöffnungsprodukte von epoxidierten Fettsäureestern mit Polyolen, Siliconöle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Trialkylphosphate sowie Mono-, Di- und/oder Tri-PEG-alkylphosphate;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE 1165574 PS** und/oder Mischester von Fettsäuren mit 6 bis 22 Kohlenstoffatomen, Methylglucose und Polyolen, vorzugsweise Glycerin,
(13) Polyalkylenglycole sowie
(14) Glycerincarbonat.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE 1943689 OS, DE 2036472 OS** und **DE 3001064 A1** sowie **EP 0077167 A1** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester Salze, besonders bevorzugt sind.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage : Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen; sowie deren Mischungen.

Als Konsistenzgeber kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -di-ester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. ein quaternierte Hydroxyethylcellulose, die unter der Bezeichnung Polymer JR 400® von Amerchol erhält-lich ist, kationische Stärke, Copolymere von Diallylammoniumsalzen und Acrylamiden, quaternierte VinylpyrrolidonNinyl-imidazol-Polymere wie z.B. Luviquat® (BASF), Kondensationsprodukte von Polyglycolen und Aminen, quaternierte Kollagenpolypeptide wie beispielsweise Lauryldimonium hydroxypropyl hydrolyzed collagen (Lamequat®L/Grünau), quaternierte Weizenpolypeptide, Polyethylenimin, kationische Siliconpolymere wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide wie z.B. beschrieben in der **FR 2252840 A1** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese, quaternierte Ammoniumsalz-Polymere wie z.B. Mirapol® A-15, Mirapol® AD-1, Mirapol® AZ-1 der Miranol.

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glucosid- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Camaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als Stabilisatoren können Metallsalze von Fettsäuren wie z.B. Magnesium-, Aluminium- und/oder Zinkstearat eingesetzt werden. Unter **biogenen Wirkstoffen** sind beispielsweise Tocopherol, Tocopherolacetat, Tocopherolpalmitat, Ascorbinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche Filmbildner sind beispielsweise quaterniertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. Typische Beispiele sind 4-Aminobenzoesäure sowie ihre Ester und Derivate (z.B. 2-Ethylhexyl-p-dimethylaminobenzoat oder p-Dimethylaminobenzoesäureoctylester), Methoxyzimtsäure und ihre Derivate (z.B. 4-Methoxyzimtsäure-2-ethylhexylester), Benzophenone (z.B. Oxybenzon, 2-Hydroxy-4-methoxybenzophenon), Dibenzoylmethane, Salicylatester, 2-Phenylbenzimidazol-5-sulfonsäure, 1-(4-tert.Butylphenyl)-3-(4'-methoxyphenyl)-propan-1,3-dion, 3-(4'-Methyl)benzylidenbornan-2-on, Methylbenzylidencampher und dergleichen. Weiterhin kommen für diesen Zweck auch feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt wer-den, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).
Zur Verbesserung des Fließverhaltens können ferner **Hydrotrope** wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche, mit 1 bis 8 Kohlenstoffen im Alkylrest wie beispielsweise Methyl- und Butylglucosid;
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose;
- Aminozucker wie beispielsweise Glucamin.

Als **Konservierungsmittel** eignen sich beispielsweise Phenoxyethanol, Formaldehydlösung, Parabene, Pentandiol oder Sorbinsäure. Als **Insekten-Repellentien** kommen N,N-Diethyl-m-touluamid, 1,2-Pentandiol oder Insect repellent 3535 in Frage, als **Selbstbräuner** eignet sich Dihydroxyaceton. Als **Treibgase** kommen Dimethylether sowie aliphatische Kohlenwasserstoffe und deren Gemische in Frage.

Als **Parfümöle** seien genannt die Extrakte von Blüten (Lavendel, Rosen, Jasmin, Neroli), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Sandel-, Guajak-, Zedem-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Moschus, Zibet und Castoreum. Als synthetische bzw. halbsynthetische Parfümöle kommen Ambroxan, Eugenol, isoeugenol, Citronellal, Hydroxycitronellal, Geraniol, Citronellol, Geranylacetat, Citral, lonon und Methylionon in Betracht.

Als **Farbstoffe** können die für kosmetische Zwecke geeigneten und zugelassenen Substanzen verwendet werden, wie sie beispielsweise in der Publikation **"Kosmetische Färbemittel" der Farbstoff** **kom-mission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Die erfindungsgemäßen Zubereitungen besitzen infolge ihres hohen Ethanolgehaltes sowie der Anwesenheit der damit verträglichen Chitosansalze zahlreiche Vorteile. So stellt man beispielsweise bei Untersuchungen an Keratinfasem fest, daß eine wäßrige Zubereitung, die 0,2 Gew.-% Chitosan Glycolate enthält, eine Biegefestigkeit von 50 % erreicht, während die gleiche Zubereitung mit 0,2 Gew.-% Chitosan Hydroxyisovaleriat und 90 Gew.-% Ethanol eine Festigung von 110 % bewirkt. Zum Vergleich: Tauscht man das Chitosan Hydroxyisovaleriat gegen einen bekannten Filmbildner vom Typ der Vinylpyrrolidon-Vinylacetat-Copolymerisate aus, beträgt die Festigung nur 60 %. In der nachfolgenden Tabelle ist die Ethanolverträglichkeit verschiedener Chitosansalze angegeben.

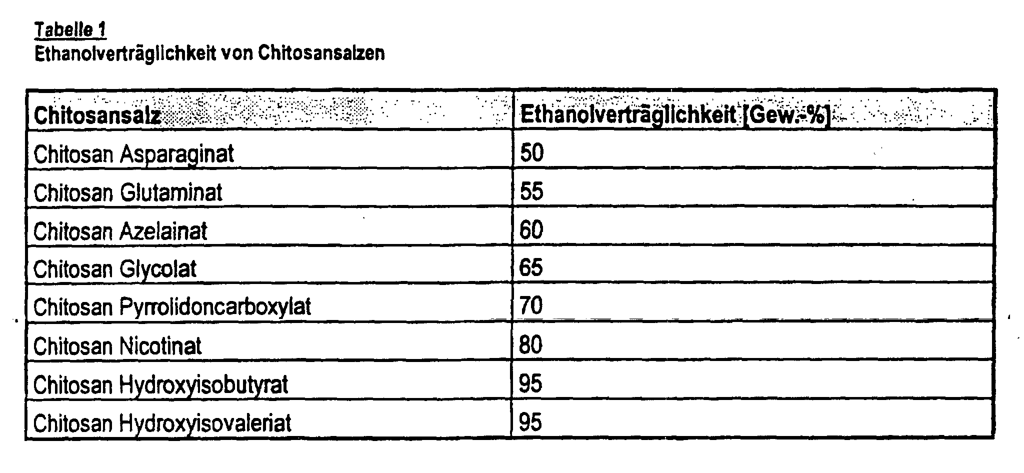

## Patentansprüche

1. Alkoholische kosmetische Zubereitungen, enthaltend
(a) 70 bis 90 Gew.-% Ethanol und
(b) 0,01 bis 5 Gew.-% Neutralisationsprodukte von Chitosan mit Pyrrolidoncarbonsäure, Nicotinsäure, Hydroxyisobuttersäure, Hydroxyisovaleriansäure und deren Gemischen,
mit der Maßgabe, daß sich die Mengenangaben mit Wasser sowie gegebenenfalls weiteren Hilfs- und Zusatzstoffen zu 100 Gew.-% ergänzen.

2. Zubereitungen nach Anspruch 1, **dadurch gekennzeichnet, daß** sie Chitosane mit einem durchschnittlichen Molekulargewicht im Bereich von 10.000 bis 1.000.000 enthalten.

3. Zubereitungen nach Anspruch 1, **dadurch gekennzeichnet, daß** sie Chitosane mit einem durchschnittlichen Molekulargewicht im Bereich von 1.000.000 bis 5.000.000 enthalten.

4. Zubereitungen nach mindestens einem der Ansprüche 1 bis 3, **dadurch gekennzeichnet, daß** sie Haarbehandlungsmittel darstellen.

5. Zubereitungen nach Anspruch 4, **dadurch gekennzeichnet, daß** sie Haarsprays darstellen.

6. Zubereitungen nach Anspruch 4, **dadurch gekennzeichnet,** sie Haargele darstellen.

7. Zubereitungen nach mindestens einem der Ansprüche 1 bis 3, **dadurch gekennzeichnet, daß** sie Deoformulierungen darstellen.

## Claims

1. Alcohol-based cosmetic preparations containing
(a) 70 to 90% by weight of ethanol and
(b) 0.01 to 5% by weight of neutralization products of chitosan with pyrrolidone carboxylic acid, nicotinic acid, hydroxyisobutyric acid, hydroxyisovaleric acid and mixtures thereof,
with the proviso that the quantities shown add up to 100% by weight with water and optionally other auxiliaries and additives.

2. Preparations as claimed in claim 1, **characterized in that** they contain chitosans with an average molecular weight of 10,000 to 1,000,000.

3. Preparations as claimed in claim 1, **characterized in that** they contain chitosans with an average molecular weight of 1,000,000 to 5,000,000.

4. Preparations as claimed in at least one of claims 1 to 3, **characterized in that** they are hair treatment compositions.

5. Preparations as claimed in claim 4, **characterized in that** they are hair sprays.

6. Preparations as claimed in claim 4, **characterized in that** they are hair gels.

7. Preparations as claimed in at least one of claims 1 to 3, **characterized in that** they are deodorant formulations.

## Revendications

1. Préparations cosmétiques alcooliques contenant
(a) 70 à 90 % en poids d'éthanol et
(b) 0,01 à 5 % en poids de produits de neutralisation de chitosane avec l'acide pyrrolidonecarboxylique, l'acide nicotinique, l'acide hydroxyisobutyrique, l'acide hydroxyisovalérique et leurs mélanges,
étant précisé que les indications quantitatives sont complétées à 100 % en poids avec de l'eau ainsi que le cas échéant avec d'autres adjuvants et additifs.

2. Préparations selon la revendication 1,
**caractérisées en ce qu'**
elles contiennent des chitosanes ayant un poids moléculaire moyen situé dans un intervalle allant de 10 000 à 1 000 000.

3. Préparations selon la revendication 1,
**caractérisées en ce qu'**
elles contiennent des chitosanes ayant un poids moléculaire moyen situé dans un intervalle allant de 1 000 000 à 5 000 000.

4. Préparations selon au moins une des revendications 1 à 3,
**caractérisées en ce qu'**
elles correspondent à des agents de traitement capillaires.

5. Préparations selon la revendication 4,
**caractérisées en ce qu'**
elles correspondent à des produits à vaporiser sur les cheveux.

6. Préparations selon la revendication 4,
**caractérisées en ce qu'**
elles correspondent à des gels capillaires.

7. Préparations selon au moins une des revendications 1 à 3,
**caractérisées en ce qu'**
elles correspondent à des formulations déodorantes.
